# EUROPEAN PATENT APPLICATION

(11) **EP 4 744 710 A1**
(43) Date of publication of application: **20.05.2026**
(21) Application number: 24213689.3
(22) Date of filing: 18.11.2024
(51) Int. Cl.: A61M 39/10, A61M 5/14

(54) **JOINT REINFORCEMENT DEVICE FOR PATIENT FLUID TRANSFER SYSTEM**

(71) Applicant: Labib, Mahmoud, Boston, MA 02134 (US); Silvaggio, Matthew Peter, Bowmanville ON L1C 1Y5 (CA)
(72) Inventor: Labib, Mahmoud, Boston, MA 02134 (US); Silvaggio, Matthew Peter, Bowmanville ON L1C 1Y5 (CA)
(74) Representative: Franke, Dirk

(57) **Abstract**

A joint reinforcement device is used in a fluid transfer system for a patient which has a fluid-receiving reservoir supported at an external location of the patient's body; a catheter insertable into the body so as to be affixed to same and for transferring fluid into and out of the patient's body; and a fluid conveyance assembly fluidically interconnecting the reservoir and the catheter. The fluid conveyance assembly has flexible components such that opposite ends of the assembly are movable relative to one another and the assembly is tensionable. Inner ends of fluidic lines forming the assembly are fluidically intercommunicated and form a joint which is not externally supported. The device comprises attachment members and an interconnecting rigid bridging member, so as to fixedly attach to the fluidic lines on either side of the joint and form an auxiliary path around the joint to transmit tensile forces along the assembly.

## Description

### FIELD OF THE INVENTION

The present invention relates generally to a fluid transfer system for transferring fluid between a patient, using a catheter, and a container or reservoir external to the patient's body, and more specifically to a device for use in such a system, which is configured to relieve tensile forces exerted on a coupling or joint between interconnected fluid-conveying components of the system.

### BACKGROUND

Many ad hoc solutions have been used by healthcare professionals to reinforce the integrity of fluid connections between medical devices and to prevent disconnections and tampering of these fluid connections. These ad hoc solutions have proven insufficient to prevent accidental disconnections, the administration of inappropriate medication through similar connector ports, and tampering from occurring.

For example, tape or dressing are an informal and commonly used solution by healthcare professionals to cover and reinforce various connection sites of a catheter system to prevent disconnections and tampering. However, this solution has proven insufficient to prevent accidental catheter disconnections, the administration of inappropriate medication through similar catheter connector ports and tampering from occurring.

### SUMMARY OF THE INVENTION

According to an aspect of the invention there is provided a joint reinforcement device for use in a fluid transfer system for a patient, wherein the fluid transfer system has:
a reservoir supported in generally fixed location externally of a body of the patient and configured to receive a fluid;
a fluid exchange catheter configured for insertion into the body of the patient so as to be fixed in relation thereto and configured for transferring the fluid between the patient and the reservoir;
a fluid conveyance assembly configured to convey the fluid between opposite ends thereof and fluidically interconnecting the reservoir and the fluid exchange catheter, wherein the fluid conveyance assembly includes first and second fluidic lines in fluidic intercommunication, wherein each of the first and second fluidic lines is configured to transfer fluid between opposite ends thereof and includes flexible tubing therebetween such that the opposite ends of the fluid conveyance assembly, which are respectively connected to the reservoir and the fluid exchange catheter, are movable relative to one another, such that the fluid conveyance assembly is tensionable;
wherein inner ones of the opposite ends of the first and second fluidic lines, which are proximal to each other and in fluidic intercommunication, form a joint which is free of external support;
wherein the joint reinforcement device comprises:
   first and second attachment members respectively configured to attach in fixed relation to the first and second fluidic lines to be positioned on either side of the joint; and
   a rigid bridging member interconnecting the first and second attachment members to form an auxiliary path around the joint for transmission of tensile forces along the fluid conveyance assembly.

According to an aspect of the invention there is provided a fluid transfer system for a patient comprising:
a reservoir supported in generally fixed location externally of a body of the patient and configured to receive a fluid;
a fluid exchange catheter configured for insertion into the body of the patient so as to be fixed in relation thereto and configured for transferring the fluid between the patient and the reservoir;
a fluid conveyance assembly configured to convey the fluid between opposite ends thereof and fluidically interconnecting the reservoir and the fluid exchange catheter, wherein the fluid conveyance assembly includes first and second fluidic lines in fluidic intercommunication, wherein each of the first and second fluidic lines is configured to transfer fluid between opposite ends thereof and includes flexible tubing therebetween such that the opposite ends of the fluid conveyance assembly, which are respectively connected to the reservoir and the fluid exchange catheter, are movable relative to one another, such that the fluid conveyance assembly is tensionable;
wherein inner ones of the opposite ends of the first and second fluidic lines, which are proximal to each other and in fluidic intercommunication, form a joint which is free of external support; and
a joint reinforcement device configured to reinforce the joint in the fluid conveyance assembly, wherein the joint reinforcement device comprises first and second attachment members attached in fixed relation to the first and second fluidic lines to be positioned on either side of the joint and a bridging member interconnecting the first and second attachment members and which is rigid, such that the joint reinforcement device forms an auxiliary path around the joint for transmission of tensile forces along the fluid conveyance assembly.

This provides an arrangement for resisting disconnection of the fluid conveyance assembly so that the fluid transfer system may function uninterruptedly.

In one arrangement, at least one of the first and second attachment members of the joint reinforcement device is configured to attach to the flexible tubing of the corresponding one of the first and second fluidic lines.

In one arrangement, when at least one of the inner ends of the first and second fluidic lines is in the form of a rigid connector, a corresponding one of the first and second attachment members of the joint reinforcement device is configured to attach to the rigid connector.

In one arrangement, at least one of the first and second attachment members of the joint reinforcement device is in the form of a clip configured to at least partially surround the corresponding one of the first and second fluidic lines at an attachment location thereto.

In one arrangement, at least one of the first and second attachment members is in the form of a spool configured to windingly receive the flexible tubing of the corresponding one of the first and second fluidic lines.

In one such arrangement, the spool includes a pair of diametrically opposite slots for releasably securing the flexible tubing to the spool.

In one arrangement, the bridging member comprises an enclosure configured to surround the j oint.

In one such arrangement, the joint reinforcement device further includes a visual indicator inside the enclosure and configured to change colour in response to contact with the fluid in the event of a leak.

In one such arrangement, the enclosure comprises first and second portions which are interconnected and movable relative to one another between an open position in which an interior of the enclosure is accessible from an exterior thereof and a closed position in which the interior is closed, wherein the enclosure further includes a lock for maintaining the first and second portions in the closed position.

In one arrangement, the bridging member comprises a fixed-length linearly-extending member and one of the first and second attachment members is supported in fixed relation to the fixed-length linearly-extending member and another one of the first and second attachment members is movably supported on the fixed-length linearly-extending member and configured to be releasably secured in fixed position to the fixed-length linearly-extending member, so as to adjust spacing between the attachment members.

In one such arrangement, the joint reinforcement device includes a ratchet mechanism operatively interconnecting said movable one of the attachment members to the fixed-length linearly-extending member and configured to resist movement of the movable attachment member away from said fixed one of the attachment members.

According to another aspect of the invention there is provided a method for transferring fluid between an external reservoir and a patient using a catheter inserted in a body of the patient, the method comprising:
fluidically interconnecting the reservoir and the catheter using a fluid conveyance assembly which is flexible such that opposite ends of the fluid conveyance assembly are movable relative to one another, wherein the fluid conveyance assembly includes a joint intermediate the opposite ends thereof so as to be free of support, wherein the joint is formed between different parts of the fluid conveyance assembly and is fluidically sealed; and
attaching a bridging device to reinforce the joint, wherein the bridging device is attached to said different parts of the fluid conveyance assembly on either side of the joint, so as to locate the bridging device in coincident relation with the j oint to provide an auxiliary path for tensile forces transmitted along the fluid conveyance assembly.

This provides an arrangement for reinforcing the joint within the fluid conveyance assembly so as to resist disconnection thereof for uninterrupted transference of the fluid between the reservoir and the patient.

In one arrangement, when the bridging device includes an enclosure, attaching a bridging device to reinforce the joint includes enclosing the joint. This may act to contain fluid in the event of a leak at the joint.

In one such arrangement, enclosing the joint includes locking the enclosure in a closed position. This may act to resist tampering of the fluid conveyance assembly at the joint in a manner that may cause disconnection.

In one arrangement, when the fluid conveyance assembly includes flexible tubing proximal to the joint and when the bridging device comprises a spool, attaching a bridging device to reinforce the joint includes wrapping a length of the flexible tubing around the spool.

In one such arrangement, the length of the flexible tubing is wrapped around the spool to leave an untensioned end portion of the flexible tubing between the joint and the spool.

In one arrangement, when the bridging device has adjustable length between opposite attachment members and comprises a ratchet mechanism operatively interconnecting the opposite attachment members for maintaining the opposite attachment members at a fixed distance, attaching a bridging device to reinforce the joint comprises attaching the opposite attachment members in spaced relation to each other on the joint and ratcheting the attachment members towards each other to relieve tension in the fluid conveyance assembly therebetween.

According to another aspect of the invention there is provided a method of reinforcing a joint in a fluid transfer system between an external reservoir and a patient, the method comprising:
providing a fluid conveyance assembly to convey fluid between the reservoir and the patient, wherein the fluid conveyance assembly is flexible such that opposite ends of the fluid conveyance assembly are movable relative to one another, wherein the fluid conveyance assembly includes a joint intermediate the opposite ends thereof so as to be free of support, wherein the joint is formed between different parts of the fluid conveyance assembly and is fluidically sealed; and
attaching a bridging device to reinforce the joint, wherein the bridging device is attached to said different parts of the fluid conveyance assembly on either side of the joint, so as to locate the bridging device in coincident relation with the j oint to provide an auxiliary path for tensile forces transmitted along the fluid conveyance assembly.

### BRIEF DESCRIPTION OF THE DRAWINGS

The invention will now be described in conjunction with the accompanying drawings in which:

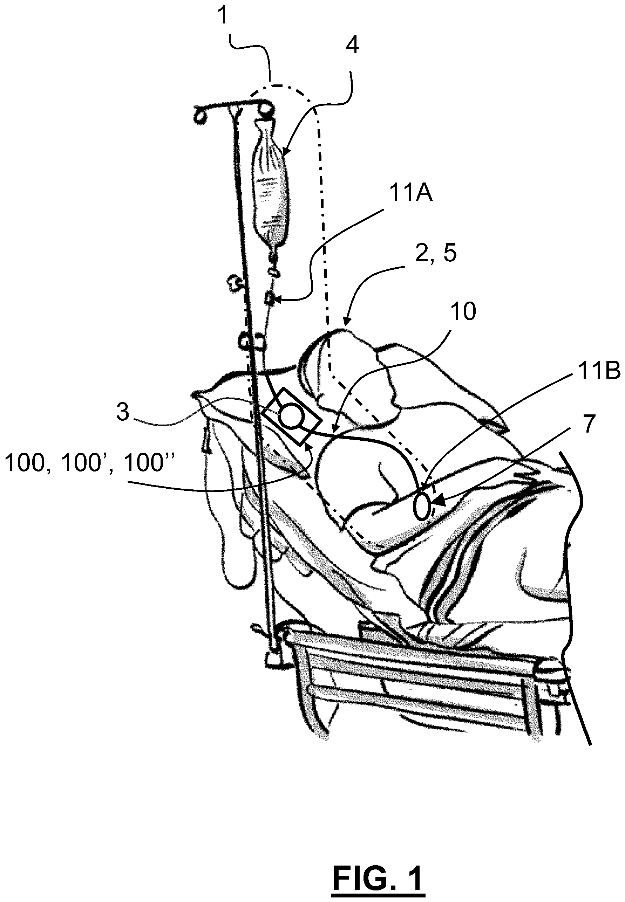

Figure 1 is a schematic diagram of a patient and a fluid transfer system connected thereto;

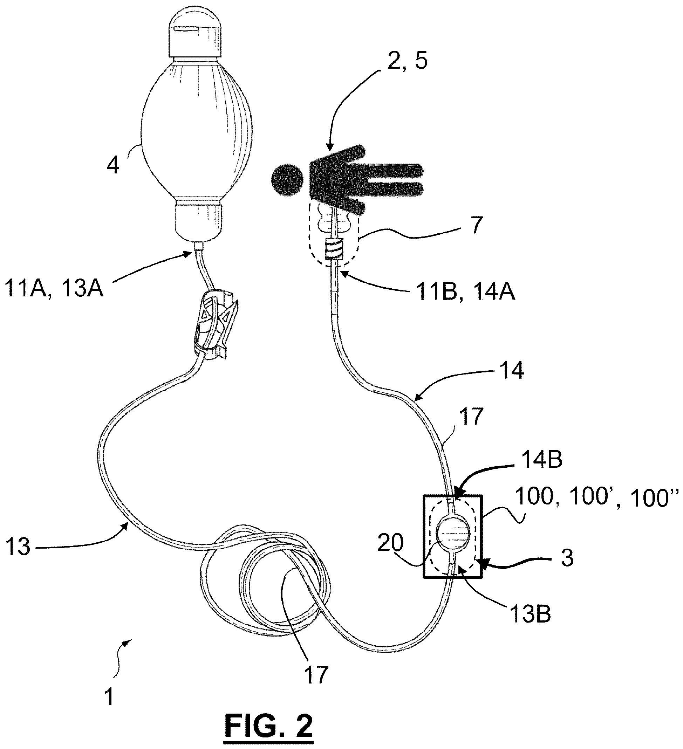

Figure 2 is another schematic diagram like Figure 1 but showing a fluid transfer system in more detail and a joint reinforcement device according to the present invention applied thereto;
Figure 3 is a perspective view of a first arrangement of joint reinforcement device according to the present invention attached or connected to a fluidic conveyance assembly;
Figure 4 is a plan view of the first arrangement of Figure 3 without a fluidic conveyance assembly;
Figure 5 is a perspective view of the first arrangement of Figure 3 shown in a closed position of an enclosure thereof;
Figure 6 is a plan view of the first arrangement of Figure 3 attached or connected to a fluidic conveyance assembly;
Figure 7 is a perspective view of a variation of the first arrangement of Figure 3;
Figure 8 is a plan view of the variation of Figure 7;
Figure 9 is a perspective view of a second arrangement of joint reinforcement device according to the present invention;
Figure 10 is another perspective view of the second arrangement of Figure 9 with attachment members thereof positioned closer together than in Figure 9;

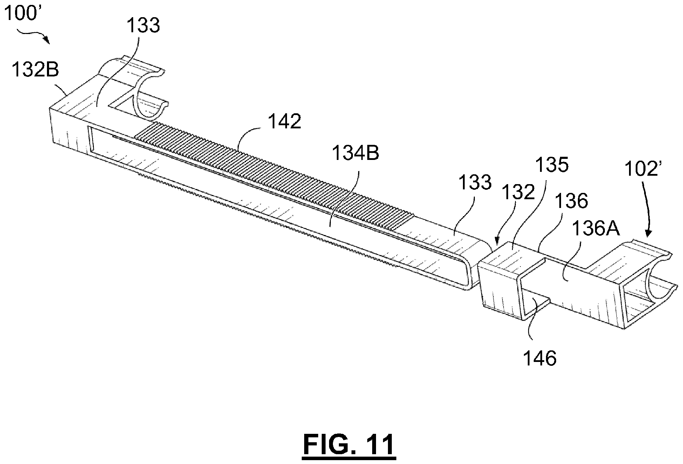

Figure 11 is an exploded perspective view of the second arrangement of Figure 9;
Figure 12 is an elevational view of the second arrangement of Figure 9 attached or connected to a fluidic conveyance assembly;
Figure 13 is a perspective view of the second arrangement with fluidic conveyance assembly as shown in Figure 12;
Figure 14 is a plan view of a variation of the second arrangement of Figure 9; and
Figure 15 is a third arrangement of joint reinforcement device according to the present invention.

In the drawings like characters of reference indicate corresponding parts in the different figures.

### DETAILED DESCRIPTION

In the accompanying figures there is shown a joint reinforcement device 100, 100' or 100" for use in a fluid transfer system 1 for a patient 2. More specifically, the joint reinforcement device acts on a fluidic joint 3 within the fluid transfer system that is normally unsupported, so as to resist disconnection of devices or lines interfacing at the joint 3, for uninterrupted operation of the fluid transfer system.

Generally speaking, the fluid transfer system 1 comprises a reservoir 4, for example in the form of a flexible pouch, supported in generally fixed location externally of a body 5 of the patient, for example on a stand which may be fixed in relation to a bed of a patient or portable so as to be movable relative to the bed, and the reservoir 4 is configured to receive a fluid, whether liquid or gaseous. Furthermore, the system 1 comprises a fluid exchange catheter 7 configured for insertion into the body of the patient so as to be fixed in relation thereto and configured for transferring the fluid within the system 1 between the patient 2, that is their body 5, and the reservoir 4. Yet further, there is a fluidic conveyance assembly 10 of the system 1, which is configured to convey the fluid between opposite ends 11A, 11B of the assembly 10 and fluidically interconnecting the reservoir 4 and the fluid exchange catheter 7.

The location of the reservoir 4 is considered generally fixed as it is intended to remain substantially constant for consistent and predictable performance of the fluid transfer system, but since the reservoir 4 itself is physically detached from the patient's body and supported at an external location thereof, the patient may inadvertently tug on the fluid conveyance assembly, in turn causing the reservoir to move or shift slightly, thereby varying its location slightly, especially if the reservoir is supported on a mobile stand as is common in hospital-type settings.

Typically, the reservoir 4 is supported at a different height relative to the patient. As such, depending on a direction of flow, that is from the reservoir to the patient or from the patient and to the reservoir, gravity may assist a transfer of the fluid therebetween.

The fluid conveyance assembly 10 includes first and second fluidic lines 13, 14 in fluidic intercommunication. Each of the first and second fluidic lines 13, 14 is configured to transfer fluid between opposite ends thereof, where the opposite ends of the first line 13 are indicated at 13A and 13B and those of the second line 14 are indicated at 14A, 14B. In some arrangements, outer ones of the opposite ends of the first and second fluidic lines 13, 14 define the opposite ends of the fluid conveyance assembly 11A and 11B. In some other arrangements, the outer end 13A of the first fluidic line 13 is fluidically connected to an intervening device, such as a filter or a pump, which in turn is fluidically connected to the reservoir 4, in which case a reservoir-side one of the opposite ends of the fluid conveyance assembly, indicated at 11A, is defined by the intervening device and not the first line's outer end 13A. Similarly, in some arrangements, the outer end 14A of the second fluidic line 14 is fluidically connected to an intervening device, such as a flow restrictor, which in turn is fluidically connected to the fluid exchange catheter 7, in which case a catheter-side one of the opposite ends of the fluid conveyance assembly, indicated at 11B, is defined by the intervening device and not the second line's outer end 14A.

Each of the first and second fluidic lines 13, 14 includes flexible tubing 17 between the respective ends of the respective one of the fluidic lines such that the opposite ends of the fluid conveyance assembly 11A and 11B, which are respectively connected to the reservoir 4 and the fluid exchange catheter 7, are movable relative to one another, such that the fluid conveyance assembly 10 is tensionable. Typically, the flexible tubing 17 interconnects the first and second ends 13A, 13B and 14A, 14B of each of the first and second fluidic lines so as to span therebetween. The opposite ends of each fluidic line are formed by rigid connectors configured to mate with corresponding connectors of different fluidic devices or components of the fluid conveyance assembly 10.

Inner ones of the opposite ends of the first and second fluidic lines 13B and 14B, which are proximal to each other and in fluidic intercommunication, form a joint 3 which is free of external support. That is, at the joint 3 there are connected different components of the fluid conveyance assembly 10 and there is an interface therebetween, which is fluidically sealed, across which fluid conveyed by the fluid transfer system flows. Since the joint 3 is free of external support, so as to be "floating," the joint 3 is movable in response to tensile forces exerted on the fluid conveyance assembly based on a location of the fluid exchange catheter 7 relative to the reservoir 4. Without external support, this may physically stress the joint and accordingly impede the fluidic seal formed thereat.

It will be appreciated that in some arrangements, the inner ends 13B, 14B of the first and second fluidic lines are fluidically interconnected, so that the fluid is transferred directly therebetween, and in other arrangements, there is an intervening device, such as a flow regulator or filter, intermediate the fluidic lines 13, 14 and to which each of the fluidic lines is respectively fluidically connected at its inner end. In the illustrated arrangement, the joint 3 is shown as including a filter 20 and auxiliary intervening fluidic couplers 22 distinct from the fluidic lines 13, 14 connected between one of the fluidic lines and the filter 20, in this case the first fluidic line 13 which is illustrated as having a longer length of tubing 17 than the second line 14.

To protect integrity of the fluidic interconnection at the j oint 3, there is provided the joint reinforcement device. Generally speaking, the joint reinforcement device such as that at 100, 100' or 100" comprises first and second attachment members 102, 102' and 103, 103' respectively configured to attach in fixed relation to the first and second fluidic lines 13, 14 to be positioned on either side of the joint 3, that is on either side of the fluidically sealed interface between distinct components which are fluidically interconnected. Furthermore, the joint reinforcement device includes a rigid bridging member 106, 106' interconnecting the first and second attachment members 102, 102' and 103, 103' to form an auxiliary path around the joint 3 for transmission of tensile forces along the fluid conveyance assembly 10. Accordingly, it will be appreciated that the j oint reinforcement device acts as a tension relief device for the j oint.

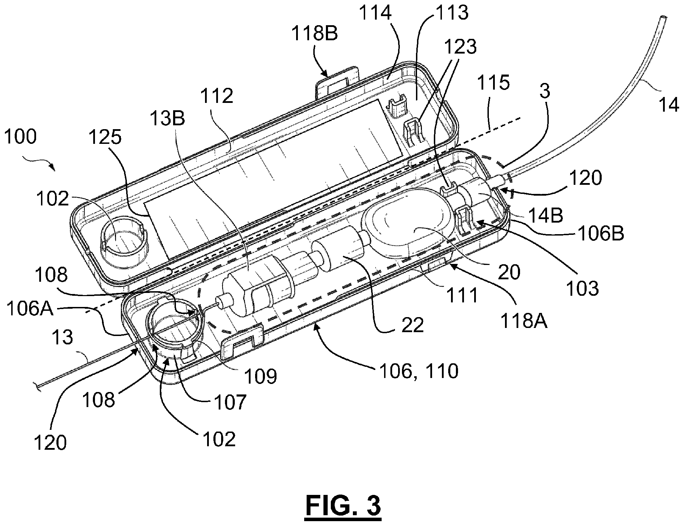

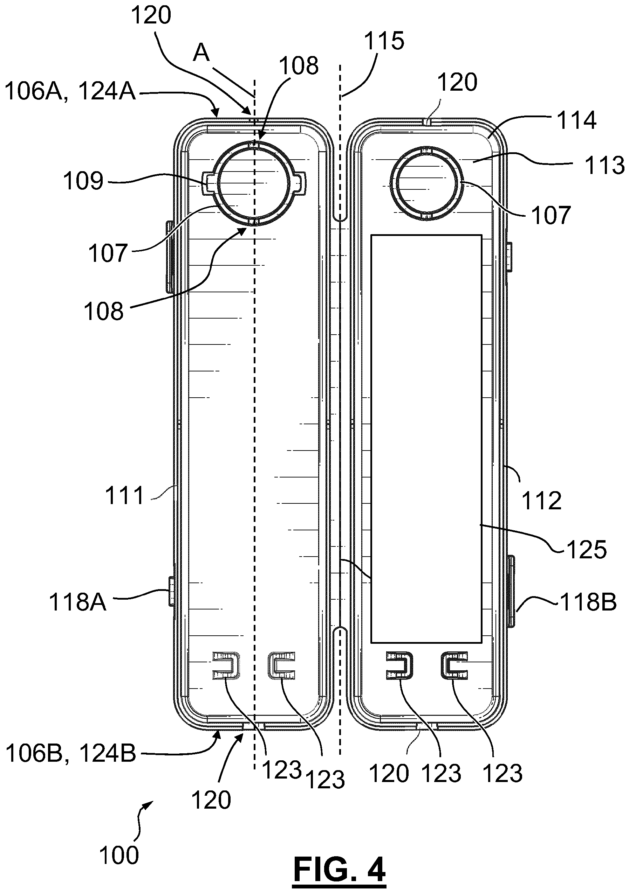

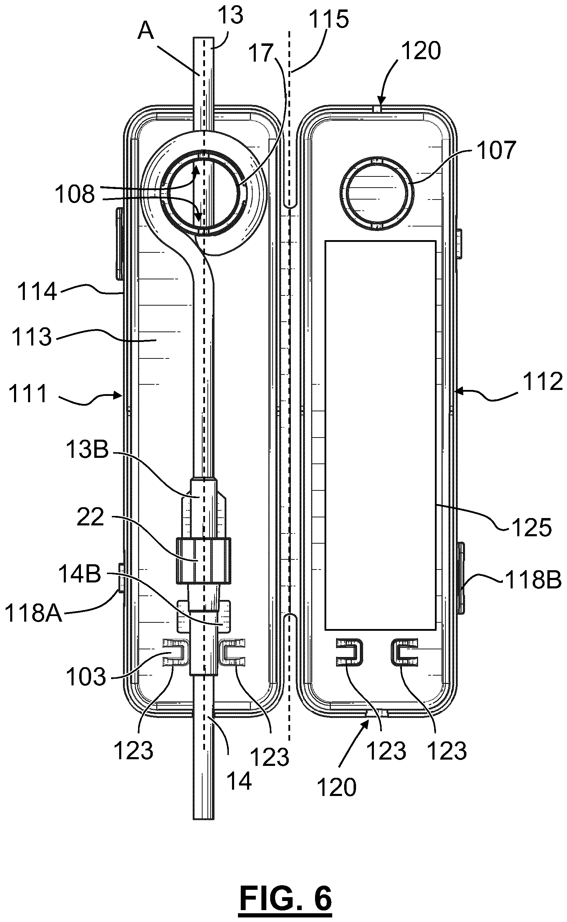

In a first illustrated arrangement of joint reinforcement device 100, as more clearly shown in Figure 3-8, which is primarily distinguished from the other illustrated arrangements by a form of the bridging member, at least one of the first and second attachment members, in this case the second one indicated at 103, is configured to attach to an endpiece of one of the first and second fluidic lines, in this case second fluidic line 14 on a catheter-side of the j oint, which endpiece defines the inner end 14B thereof, and which is in the form of a rigid connector. Thus, such an attachment member is generally in the form of a clip configured to at least partially surround the corresponding fluidic line at an attachment location thereto.

In the first illustrated arrangement 100, another one of the first and second attachment members, in this case the first one indicated at 102, is in the form of a spool configured to windingly receive the flexible tubing of the corresponding one of the first and second fluidic lines, in this case first fluidic line 13 which is on a reservoir-side of the joint 3 so as to be proximal to same in comparison to the other one of the fluidic lines 14. In the illustrated arrangement, the spool is in the form of a hollow circular cylindrical wall 107 upstanding from the bridging member 106. Furthermore, the spool includes a pair of diametrically opposite slots 108 for releasably securing the flexible tubing to the spool. More specifically, the slots 108 are in the form of cut-outs open at a distal end of the spool relative to bridging member 106 carrying the same, and the slots are coincident with an axis A, so as to be coaxial therewith, defined by the bridging member 106 which is linearly-extending between longitudinally opposite ends 106A, 106B. Furthermore, the spool carries retention elements 109 in the form of outwardly projecting tabs for maintaining wound tubing on the wall of the spool.

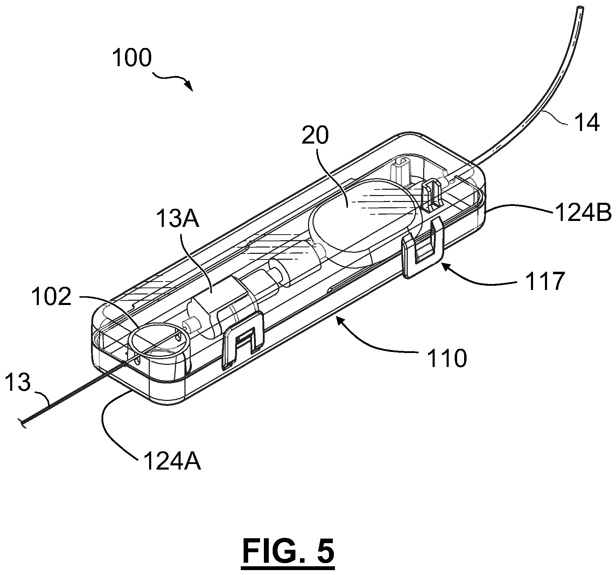

The bridging member 106 in the first arrangement comprises an enclosure 110 configured to surround the joint. The enclosure 110 comprises first and second portions 111, 112 which collectively provide a container, in this case portion 111, and a lid therefor, in this case portion 112, to close an interior of the container 111. In the illustrated arrangement, the lid 112 is container-shaped so as to partially form a total volume of the enclosure in the closed position. That is, each portion comprises a base wall 113, which is substantially planar, and a peripheral wall 114 delimiting an interior over the base wall. The first and second portions 111, 112 are interconnected and movable relative to one another between an open position, in which an interior of the enclosure is accessible from an exterior thereof, as shown for example in Figure 3, and a closed position in which the interior is closed, as shown for example in Figure 5. More specifically, the first and second portions 111, 112 are hingedly interconnected for relative movement about a pivot axis 115 along a common side of the first and second portions. The enclosure 110 further includes a lock 117 for maintaining the first and second portions 111, 112 in the closed position. Each of the portions carries a component of the lock, namely a catch 118A and a latch 118B, to securably mate in the closed position of the enclosure.

To permit passage of the fluidic lines 13, 14 into the enclosure in the closed position, at least one or both of the first and second portions 111, 112 include a cut-out 120 which cooperates with a registering portion of the other one of the enclosure portions in the closed position to form an opening into the enclosure 110.

Thus, the clip-type attachment member 103 of the first illustrated arrangement is formed by a pair of generally parallel legs 123 which are deflectable away from each other or are resiliently deformable to snugly receive and retain the fluidic line. The legs 123 project from the container portion 111 at its base wall and are interconnected thereby, such that the container portion 111 may be considered to form part of the clip, at least that portion adjacent and intermediate the legs.

Since the first and second portion 111, 112 of the enclosure are mirror images of the other, each carries cooperating features that collectively form the attachment members and bridging member in the closed position, as well as the apertures or openings in axially opposite ends 124A and 124B of the enclosure 110 which define the ends 106A, 106B.

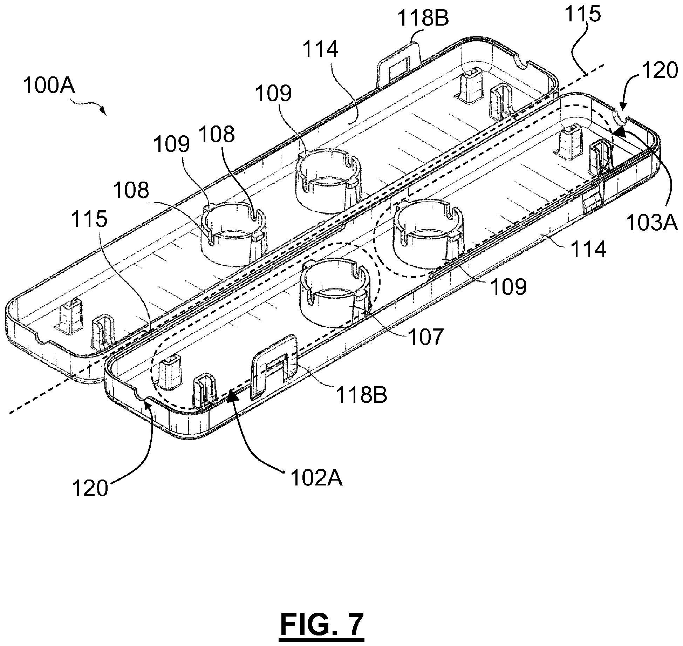

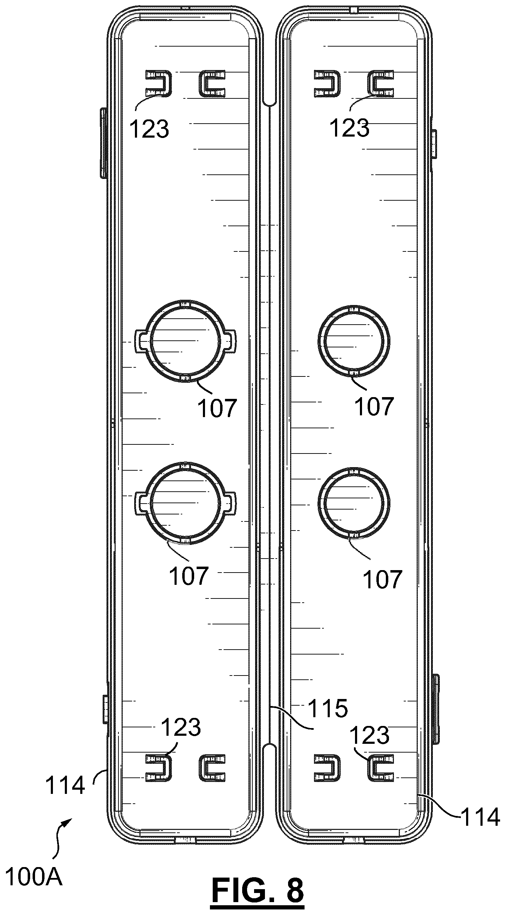

Furthermore, in a variation 100A of the first arrangement shown in Figures 7 and 8, each attachment member 102A, 103A comprises an assembly of a clip and a spool each of which supports one of the fluidic lines 13, 14. Generally speaking, the spools are supported in adjacent relation to each other within the enclosure, and the clips are supported in distal relation thereto, outwardly of the spools. This arrangement may be suited for reinforcing a joint which has an intervening component between the fluidic lines 13, 14 and having lengths of flexible tubing on either end of the intervening component. It will be appreciated that, in this variation, the form of the distinct attachment members of a common one of the joint reinforcement device is the same, unlike the earlier illustrated arrangement in which the attachment members had different form.

Further to the attachment members and the bridging member, the joint reinforcement device of the first arrangement includes a visual indicator 125 (schematically shown) inside the enclosure 110 and configured to change colour in response to contact with the fluid in the event of a leak. The visual indicator 125 may comprise a paper-like substrate in the form of a sheet lining at least a portion of the interior of the enclosure, such as the base wall of one of the portions of the enclosure, which is shown to be the lid portion 112. Thus, initially, the enclosure 110 acts to contain a potential leak at the joint, the presence of which may be visually demonstrated or signalled to a user via the visual indicator 125.

A second illustrated arrangement 100' of joint reinforcement device indicated at 100' comprises at least one of the first and second attachment members in the form of a clip and configured to attach to the flexible tubing 17 of the corresponding one of the first and second fluidic lines. More specifically, both attachment members 102', 103' are in the form of clips. Each clip is configured to at least partially surround the corresponding fluidic line at an attachment location thereto. More specifically, each of the clips is in the form of a C-shaped length of channel 128 with resiliently deformable end portions 129, defining ends of the 'C', to permit passage of the fluidic line in a transverse direction to a longitudinal direction of the respective attachment clip. In the second illustrated arrangement, opposite sidewalls of the short C-channels 102' and 103' are curved so as to define a generally circular interior cross-section suited for matably receiving a circular cylindrical member therein.

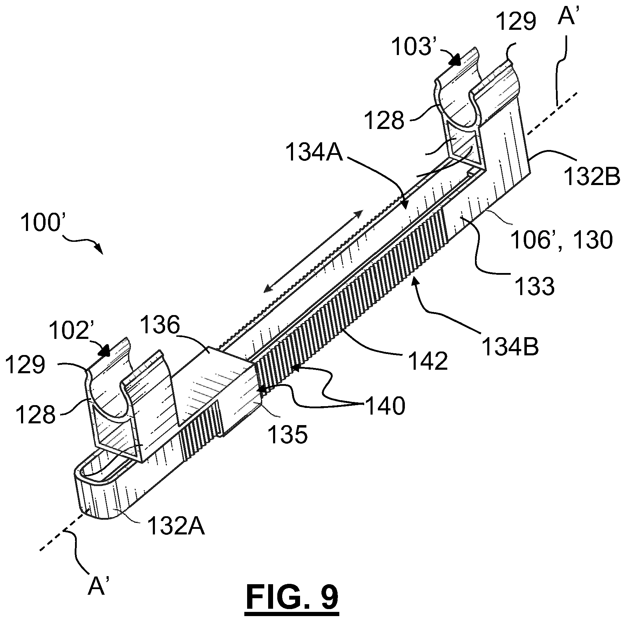

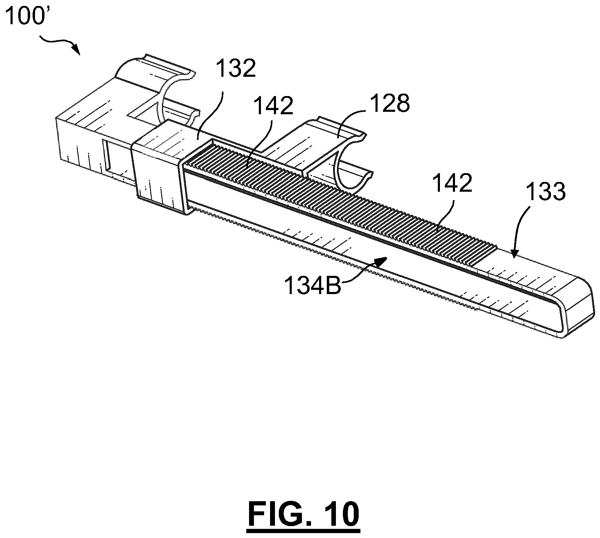

In the second arrangement 100', the bridging member 106' comprises a fixed-length linearly-extending member 130 spanning between opposite ends 132A, 132B thereof, and one of the first and second attachment members, such as 103', is supported in fixed relation to the fixed-length member 130, so as to form a fixed attachment member, and another one of the first and second attachment members, such as 102', is movably supported on the fixed-length member, so as to form a movable or adjustable attachment member, and configured to be releasably secured in fixed position to the fixed-length linearly-extending member, so as to adjust spacing between the attachment members, as represented by Figures 9-10. Thus, the bridging member 106' additionally includes a carrier 132 supporting the movable attachment member 102' and operatively connected to the fixed-length member 130 for movement relative thereto, for example by a ratchet mechanism as described further below.

In the illustrated arrangement, the fixed-length member 130 is tubular, so as to be hollow, but in a transverse direction to its length between opposite ends 132A, 132B and width between opposite sides 133, such that the ends 132A, 132B are closed and a top side 134A and underside 134B are open. The opposite ends 132A, 132B of the fixed-length member define the ends of the bridging member 106' of the second illustrated arrangement. Furthermore, it will be appreciated that the fixed attachment member 103' is disposed at one of the ends, in this case 132B, of the fixed-length member 130 of the bridging member 106'.

The carrier 132 is repositionable in a longitudinal direction of the fixed-length member 130 at one of a plurality of positions, which are predetermined when a ratchet interconnection mechanism is provided. In the illustrated arrangement 100', the carrier 132 comprises an annular or tubular collar 135 encompassing the fixed-length member along its axis A', so that it is axially displaceable relative to the fixed-length member. Integrally formed with the collar, so as to be attached thereto, is a support base 136 of the carrier 132, which is substantially planar and defining a skid surface 136A on its underside for sliding engagement with the top side 134A of the fixed-length member, defined by laterally opposite top edges, so as to be configured to slidably rest on the top side of the fixed-length member 130 to support the attachment member 102' in a prescribed orientation relative to the fixed attachment member 103'. The skid surface 136A is registered with a top of the collar 135 so as to be coplanar therewith. Also, it will be appreciated that the carrier 132 is longitudinally elongated between a first end defined by the collar 135 and a second end at which the attachment member 102' is disposed.

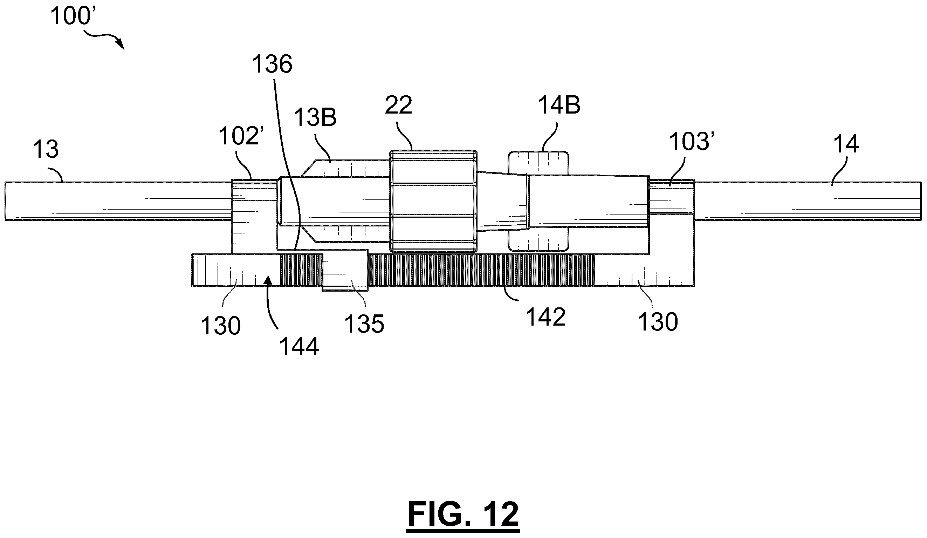

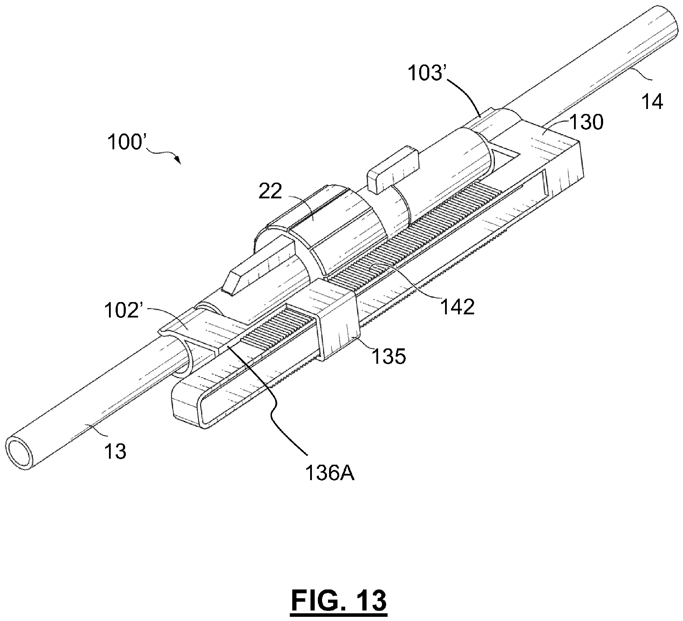

For releasably securing the movable attachment member 102' at a fixed position relative to the fixed attachment member, the joint reinforcement device 100' includes a ratchet mechanism 140 operatively interconnecting the movable attachment member to the bridging member and configured to resist movement of the movable attachment member away from the fixed attachment member. More specifically, the ratchet mechanism 140 is provided by a plurality of parallel ridges 142 on laterally opposite sides 133 of the fixed-length member 130, which extend in a transverse direction to both the lateral direction and the longitudinal direction of the member 130, from the underside 134B to the top side 134A of the fixed-length member 130. Between adjacent pairs of ridges are grooves which act as detents or stops defining a plurality of predetermined positions of the movable attachment member 102' relative to the fixedly located or stationary attachment member 103'. The carrier 132 supporting the movable attachment member 102', which forms part of the bridging member 106', carries one or more cooperating ridges or teeth (not shown) on its inner diametrically opposite surfaces 146 arranged to face the ridges 142 when the carrier is operatively connected to the fixed-length member as shown in Figures 9-10 and 12-13. To reposition the movable member 102' further from the fixed member 103' so as to increase the spacing or distance therebetween, the fixed-length member 130 is resiliently deformable in a lateral direction between its sides 133 so as to release the movable attachment member. Similarly, the collar 135 may be resiliently deformable, but otherwise substantially rigid to maintain its shape and intermeshing engagement of the ratchet mechanism, so as to be displaceable longitudinally of the fixed-length member 130 while in intermeshing engagement with the teeth 142.

In other words, the movable attachment member 102' is carried on the fixed-length member 130 by a substantially rigid annulus and configured to matably slide longitudinally of the fixed-length member. The annulus is configured to be releasably secured to the fixed-length member by diametrically opposite inwardly-projecting elements which are configured to be received in the detents. Also, the annulus is resiliently deformable in a direction directed between the inward projections, so as to slide over the guide surfaces of the teeth.

In the second illustrated arrangement, the bridging member 106' does not encapsulate or otherwise surround any portion of the joint or, more generally, the fluid conveyance assembly. Thus, there is no ability of the second arrangement 100' to contain any leaks in the j oint.

In the unenclosed ratchet-type arrangement of joint reinforcement device, the attachment members 102', 103' are attached to the fluidic lines in immediately adjacent relation to the rigid connectors defining the ends 13B, 14B of the first and second fluidic lines, such that although the clips attach to the flexible tubing 17, the attachment members 102' and 103' act to abut distal ends of the rigid connectors (relative to their terminuses) to retain the joint 3 as interconnected by disposing the interconnecting components of the fluidic conveyance assembly in sandwiched relation between the attachment members 102' and 103'.

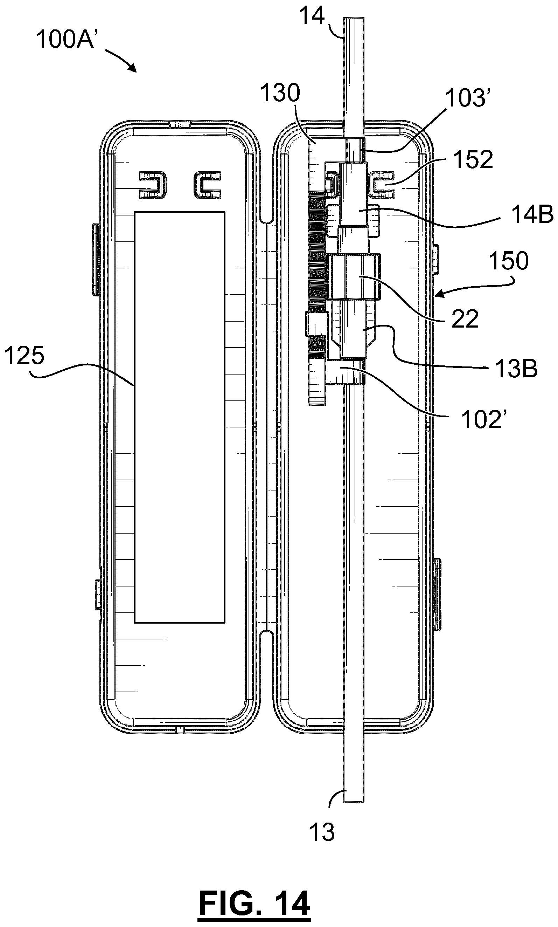

A variation 100A' of the second arrangement is shown in Figure 14 and includes a surrounding enclosure 150. The enclosure 150 is similar to that previously indicated at 110 except that it carries only one attachment member 152 for physically attaching to the fluidic conveyance assembly, in this case that of the clip type. The single attachment member 152 acts to position the enclosure in fixed relation to the fluid conveyance assembly, in a manner registered with the joint, so that any potential leaks can be contained when the enclosure 150 is in a closed position similar to that shown in Figure 5.

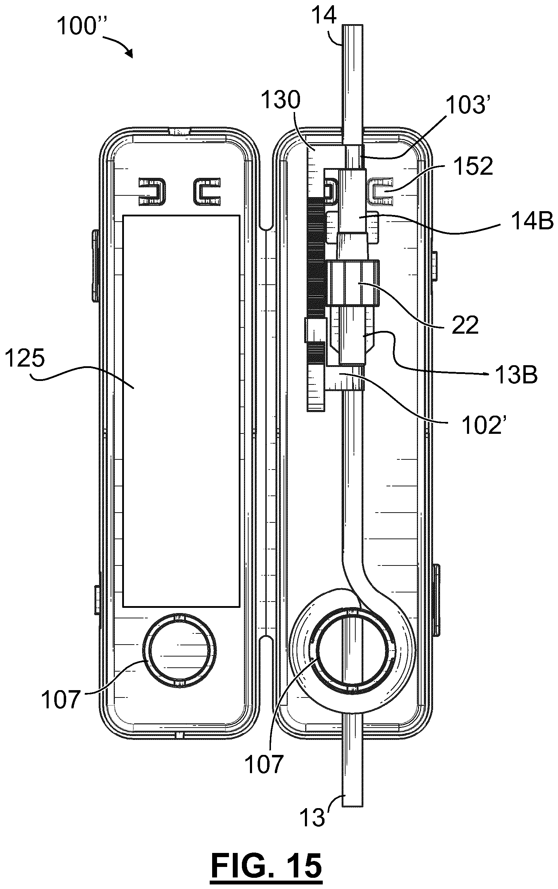

A third arrangement 100" of joint reinforcement device comprises an assembly of the enclosure-type device 100 and the ratchet-type device 100'. As shown in Figure 15, the enclosure-type device has two attachment members of different types connecting to different components of the fluidic conveyance assembly 10. Accordingly, the combination device has two independent bridging members. When the enclosure-type device has an attachment member in the form of a clip configured to receive a rigid connector of one of the fluidic lines, then the devices 100, 100' are arranged relative to the joint in an offset configuration. That is, the ratchet-type device connects on an outer side of the clip, between the wall 114 of the respective portion of the enclosure and the clip-type attachment member, and the other attachment member of device 100' is disposed intermediate the clip and the spool. As such, the two types of bridging members of such a combination device may act to provide substantially parallel bypass paths for tensile forces around the joint.

The foregoing provide arrangements for resisting disconnection of the fluid conveyance assembly so that the fluid transfer system may function uninterruptedly.

There is also disclosed herein a method for transferring fluid between an external reservoir and a patient using a catheter inserted in a body of the patient, as well as a method of reinforcing a joint in fluid transfer system formed between the external reservoir and the patient.

Initial steps of the methods include, in the case of the method of reinforcing the joint, a step of providing a fluid conveyance assembly 10 to convey fluid between the reservoir 4 and the patient 2, or in the case of the method of transferring fluid, a step of fluidically interconnecting the reservoir 4 and the catheter 7 using a fluid conveyance assembly 10, which assembly is flexible such that opposite ends 13A, 13B of the fluid conveyance assembly are movable relative to one another. The provided fluid conveyance assembly includes a joint 3 intermediate the opposite ends thereof so as to be free of support, and the joint 3 is formed between different parts of the fluid conveyance assembly and is fluidically sealed.

Afterwards, both methods include a step of attaching a bridging device such as 100, 100' or 100" to reinforce the joint 3. The bridging device is attached to the different parts of the fluid conveyance assembly on either side of the joint, so as to locate the bridging device in coincident relation with the joint to provide an auxiliary path for tensile forces transmitted along the fluid conveyance assembly.

When the bridging device includes an enclosure such as that indicated at 110 or 150, the step of attaching the bridging device to the joint 3 includes enclosing the joint. This may act to contain fluid in the event of a leak at the joint. Furthermore, enclosing the joint preferably includes locking the enclosure in a closed position, such as via elements 118A and 118B which collectively form a lock 117. This may act to resist tampering of the fluid conveyance assembly at the joint in a manner that may cause disconnection.

When the fluid conveyance assembly 10 includes flexible tubing 17 proximal to the joint 3 and when the bridging device comprises a spool 107, the step of attaching the bridging device typically includes wrapping a length of the flexible tubing around the spool. The length of the flexible tubing is wrapped around the spool to leave an untensioned end portion of the flexible tubing between the joint and the spool. The end portion of the flexible tubing usually supports a rigid endpiece for connecting to other components of the fluidic conveyance assembly.

When the bridging device has adjustable length between opposite attachment members such as 102', 103' and comprises a ratchet mechanism 140 operatively interconnecting the opposite attachment members for maintaining the opposite attachment members at a fixed distance, the step of attaching the bridging device comprises attaching the opposite attachment members in spaced relation to each other on the joint 3 and ratcheting the attachment members towards each other to relieve tension in the fluid conveyance assembly therebetween.

This provides an arrangement for reinforcing the joint within the fluid conveyance assembly so as to resist disconnection thereof for uninterrupted transference of the fluid between the reservoir and the patient.

As described hereinbefore, the present invention relates to a joint reinforcement device used in a fluid transfer system for a patient which has a fluid-receiving reservoir supported at an external location of the patient's body; a catheter insertable into the body so as to be affixed to same and for transferring fluid into and out of the patient's body; and a fluid conveyance assembly fluidically interconnecting the reservoir and the catheter. The fluid conveyance assembly has flexible components such that opposite ends of the assembly are movable relative to one another and the assembly is tensionable. Inner ends of fluidic lines forming the assembly are fluidically intercommunicated and form a joint which is not externally supported. The device comprises attachment members and an interconnecting rigid bridging member, so as to fixedly attach to the fluidic lines on either side of the j oint and form an auxiliary path around the joint to transmit tensile forces along the assembly.

The scope of the claims should not be limited by the preferred embodiments set forth in the examples but should be given the broadest interpretation consistent with the specification as a whole.

## Claims

1. A fluid transfer system for a patient comprising:
a reservoir supported in generally fixed location externally of a body of the patient and configured to receive a fluid;
a fluid exchange catheter configured for insertion into the body of the patient so as to be fixed in relation thereto and configured for transferring the fluid between the patient and the reservoir;
a fluid conveyance assembly configured to convey the fluid between opposite ends thereof and fluidically interconnecting the reservoir and the fluid exchange catheter, wherein the fluid conveyance assembly includes first and second fluidic lines in fluidic intercommunication, wherein each of the first and second fluidic lines is configured to transfer fluid between opposite ends thereof and includes flexible tubing therebetween such that the opposite ends of the fluid conveyance assembly, which are respectively connected to the reservoir and the fluid exchange catheter, are movable relative to one another, such that the fluid conveyance assembly is tensionable;
wherein inner ones of the opposite ends of the first and second fluidic lines, which are proximal to each other and in fluidic intercommunication, form a joint which is free of external support; and
a joint reinforcement device configured to reinforce the joint in the fluid conveyance assembly, wherein the joint reinforcement device comprises first and second attachment members attached in fixed relation to the first and second fluidic lines to be positioned on either side of the joint and a bridging member interconnecting the first and second attachment members and which is rigid, such that the joint reinforcement device forms an auxiliary path around the joint for transmission of tensile forces along the fluid conveyance assembly.

2. The fluid transfer system of claim 1 wherein at least one of the first and second attachment members of the joint reinforcement device is configured to attach to the flexible tubing of the corresponding one of the first and second fluidic lines.

3. The fluid transfer system of claim 1 or 2 wherein, when at least one of the inner ends of the first and second fluidic lines is in the form of a rigid connector, a corresponding one of the first and second attachment members of the joint reinforcement device is configured to attach to the rigid connector.

4. The fluid transfer system of any one of claims 1 to 3 wherein at least one of the first and second attachment members of the joint reinforcement device is in the form of a clip configured to at least partially surround the corresponding one of the first and second fluidic lines at an attachment location thereto.

5. The fluid transfer system of any one of claims 1 to 4 wherein at least one of the first and second attachment members is in the form of a spool configured to windingly receive the flexible tubing of the corresponding one of the first and second fluidic lines.

6. The fluid transfer system of claim 5 wherein the spool includes a pair of diametrically opposite slots for releasably securing the flexible tubing to the spool.

7. The fluid transfer system of any one of claims 1 to 6 wherein the bridging member comprises an enclosure configured to surround the joint.

8. The fluid transfer system of claim 7 wherein the joint reinforcement device further includes a visual indicator inside the enclosure and configured to change colour in response to contact with the fluid in the event of a leak.

9. The fluid transfer system of claim 7 or 8 wherein the enclosure comprises first and second portions which are interconnected and movable relative to one another between an open position in which an interior of the enclosure is accessible from an exterior thereof and a closed position in which the interior is closed, wherein the enclosure further includes a lock for maintaining the first and second portions in the closed position.

10. The fluid transfer system of any one of claims 1 to 9 wherein the bridging member comprises a fixed-length linearly-extending member and one of the first and second attachment members is supported in fixed relation to the fixed-length linearly-extending member and another one of the first and second attachment members is movably supported on the fixed-length linearly-extending member and configured to be releasably secured in fixed position thereto, so as to adjust spacing between the attachment members.

11. The fluid transfer system of claim 10 wherein the joint reinforcement device includes a ratchet mechanism operatively interconnecting said movable one of the attachment members to the fixed-length linearly-extending member and configured to resist movement of the movable attachment member away from said fixed one of the attachment members.

12. A method for transferring fluid between an external reservoir and a patient using a catheter inserted in a body of the patient, the method comprising:
fluidically interconnecting the reservoir and the catheter using a fluid conveyance assembly which is flexible such that opposite ends of the fluid conveyance assembly are movable relative to one another, wherein the fluid conveyance assembly includes a joint intermediate the opposite ends thereof so as to be free of support, wherein the joint is formed between different parts of the fluid conveyance assembly and is fluidically sealed; and
attaching a bridging device to reinforce the joint, wherein the bridging device is attached to said different parts of the fluid conveyance assembly on either side of the joint, so as to locate the bridging device in coincident relation with the j oint to provide an auxiliary path for tensile forces transmitted along the fluid conveyance assembly.

13. The method of claim 12 wherein, when the bridging device includes an enclosure, attaching a bridging device to reinforce the j oint includes enclosing the j oint.

14. The method of claim 12 or claim 13 wherein, when the fluid conveyance assembly includes flexible tubing proximal to the joint and when the bridging device comprises a spool, attaching a bridging device to reinforce the joint includes wrapping a length of the flexible tubing around the spool.

15. The method of any one of claims 12 to 14 wherein, when the bridging device has adjustable length between opposite attachment members and comprises a ratchet mechanism operatively interconnecting the opposite attachment members for maintaining the opposite attachment members at a fixed distance, attaching a bridging device to reinforce the joint comprises attaching the opposite attachment members in spaced relation to each other on the joint and ratcheting the attachment members towards each other to relieve tension in the fluid conveyance assembly therebetween.
